# EUROPEAN PATENT SPECIFICATION

(11) **EP 0 468 448 B1**
(45) Date of publication and mention of the grant of the patent: **12.06.1996**
(21) Application number: 91112331.3
(22) Date of filing: 23.07.1991
(51) Int. Cl.: A61K 38/55, C07K 2/00

(54) **Analogs of hirudin having antiplatelet activity**
Hirudin-Analoge mit Antiblutplättchen-Aktivität
Analogues de l'hirudine possédant une activité antiplaquettaire

(30) Priority: 24.07.1990 US 557289
(43) Date of publication of application: 29.01.1992
(73) Proprietor: MERRELL PHARMACEUTICALS INC., Cincinnati, Ohio 45215-6300 (US)
(72) Inventor: Krstenansky , John L., Palo Alto, California 94306 (US); Broersma, Robert J., Jr., Noblesville, Indiana 46060 (US)
(74) Representative: VOSSIUS & PARTNER

(56) References cited:
- EP-A- 0 276 014
- EP-A- 0 332 523
- EP-A- 0 333 356
- EP-A- 0 341 607
- EP-A- 0 372 503
- EP-A- 0 421 366
- EP-A- 0 421 367

## Description

This invention relates to the use of peptide analogs for the preparation of pharmaceutical compositions for use as anticoagulant and antiplatelet agents.

Anticoagulants are useful therapeutic agents in the pharmacological treatment of, for example, acute deep venous thrombosis, pulmonary embolism, acute arterial embolization of the extremities, myocardial infarction, and disseminated intravascular coagulation. Prophylactic administration of anticoagulants is believed to prevent a recurrence of embolism in patients with rheumatic or arteriosclerotic heart disease and to prevent certain thromboembolic complications of surgery. Administration of anticoagulants has also been indicated in the treatment of coronary artery and cerebrovascular disease. Arterial thrombosis, particularly in arteries supplying the heart muscle and brain, is a leading cause of death.

Antiplatelet agents are useful therapeutic agents in the pharmacological treatment of those platelet associated thromboembolic diseases that are primarily arterial in origin. For example, antiplatelet agents can be used to prevent reoccurence myocardial infarction and strokes.

Hirudin is a 65 residue polypeptide isolated from the salivary glands of leeches. It is an anticoagulant agent, which is a thrombin specific inhibitor. Although quite potent, clinical use of hirudin isolated from leech extracts seems unlikely because of its limited quantity, expense and allergic reactions which commonly follow administration of any foreign protein of this size.

Applicant has previously discovered a specific region of hirudin that is responsible, at least in part, for its anticoagulant activity. This region has been chemically synthesized and certain of its analogs appear to bind to the recognition site of thrombin but not the enzymatic cleavage site which is spatially separate. Binding of the synthetic peptides competitively prevents binding of the fibrinogen to the recognition site of thrombin, a prerequisite to fibrin production and clot formation.

Several reports have described the ability of the oligopeptide Arg-Gly-Asp and related peptides to inhibit the platelet-dependent thrombus formation. Y. Cadroy, et al., J. Clin. Invest. 84, 939-944 (1989).

Peptide derivatives of the formula 1

X-A₁A₂A₃A₄A₅A₆A₇A₈A₉A₁₀A₁₁-Y (1)

wherein
- X: is an amino terminal residue selected from hydrogen, one or two alkyl groups of from 1 to 6 carbon atoms, one or two acyl groups of from 2 to 10 carbon atoms, carbobenzyloxy, H₂NC(NH)-, or t-butyloxy carbonyl group;
- A₁: is a bond or is a peptide containing from 1 to 11 residues of any amino acid;
- A₂: is a group of one of the the formula 6 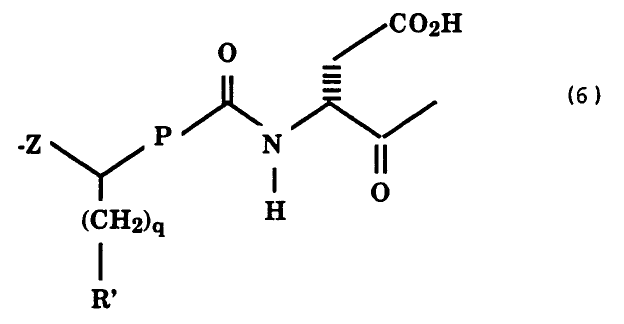
wherein
- Z: is a bond or a -NH- or -N(C₁-C₄alkyl)-group;
- q: is O or an integer of from 1 to 5;
- R': is -NH₂ or -N(H)C(=NH)NH₂;
- P: is an ortho-, meta-, or para- phenylene or a 1,2-, 1,3-, or 1,4-cyclohexadiyl;
- A₃: is Phe, SubPhe, β-(2- and 3-thienyl)alanine, β-(2-and 3-furanyl)alanine, β-(2-, 3-, and 4-pyridyl)alanine, β-(benzothienyl-2- and 3-yl)alanine, β-(1- and 2-naphthyl)alanine, Tyr or Trp;
- A₄: is Glu, Asp, Ser(OSO₃H), Ser(OPO₃H), hSer(OSO₃H), cysteic acid or homocysteic acid;
- A₅: is any amino acid;
- A₆: is Ile, Val, Leu, Nle, or Phe;
- A₇: is Pro, Hyp, 3,4-dehydroPro, thiazolidine-4-carboxylate, Sar, NMePgl or D-Ala;
- A₈: is any amino acid;
- A₉: is any amino acid;
- A₁₀: is a lipophilic amino acid selected from Tyr, Trp, Phe, Leu, Nle, Ile, Val, Cha and Pro or is a dipeptide containing at least one of these lipophilic amino acids;
- A₁₁: is a bond or is a peptide fragment containing from one to five residues of any amino acid; and
- Y: is a carboxy terminal residue selected from OH, C₁-C₆ alkoxy, amino, mono- or di-(C₁-C₄) alkyl substituted amino, or benzylamino;
and the pharmaceutically acceptable salts may be used for the preparation of pharmaceutical compositions for use as anticoagulant agents.

The following common abbreviations of the amino acids are used throughout this specification:
- Gly -: glycine
- Ala -: alanine
- Val -: valine
- Leu -: leucine
- Ile -: isoleucine
- Cha -: cyclohexylalanine
- Orn -: ornithine
- Pro -: proline
- Phe -: phenylalanine
- Trp -: tryptophan
- Met -: methionine
- Ser -: serine
- Thr -: threonine
- Cys -: cysteine
- Tyr -: tyrosine
- Asn -: asparagine
- Gln -: glutamine
- Asp -: aspartic acid
- Glu -: glutaminc acid
- Lys -: lysine
- Hly -: homolysine
- Arg -: arginine
- Har -: homoarginine
- His -: histidine
- Nle -: norleucine
- Hyp -: hydroxyproline
- Glt -: glutaryl
- Mal -: maleyl
- Npa -: β-(2-naphthyl)alanine
- 3,4-dehydroPro -: 3,4-dehydroproline
- Tyr(SO₃H) -: tyrosine sulfate
- Pgl -: phenylglycine
- NMePgl -: N-methyl-phenylglycine
- Sar -: sarcosine (N-methylglycine)
- pSubPhe -: para substituted phenylalanine
- SubPhe -: ortho, meta, or para, mono- or di- substituted phenylalanine
- DAla -: D-alanine
- Ac -: acetyl
- Suc -: succinyl
- pClPhe -: para-chloro-phenylalanine
- pNO₂Phe -: para-nitro-phenylalanine
- Tyr(Me) -: O'-methyl-4-tyrosine
- 5GP -: 5-guanidinopentyl
- 5AP -: 5-aminopentyl
An alkyl group and the alkyl portion of an alkoxy group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl, succinyl, maleyl, and glutaryl. A halogen group is a fluoro, chloro, bromo or iodo group.

The term "any amino acid" as used herein includes the naturally occurring amino acids as well as other "non-protein" α-amino acids commonly utilized by those in the peptide chemistry art when preparing synthetic analogs of naturally occurring peptides. The naturally occurring amino acids are glycine, alanine, valine, leucine,isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of "non-protein" α-amino acids are norleucine, norvaline, alloisoleucine, homoarginine, thiaproline, dehydroproline, hydroxyproline (Hyp), homoserine, Ser(OSO₃H), hSer(OSO₃H), cysteic acid, homocysteic acid, cyclohexylglycine (Chg), α-amino-n-butyric acid (Aba), cyclohexylalanine (Cha), aminophenylbutyric acid (Pba), phenylalanines substituted at the ortho, meta, or paraposition of the phenyl moiety with one or two of the following, a (C₁-C₄) alkyl, (C₁-C₄) alkoxy, halogen, or nitro groups or substituted with a methylenedioxy group, β-2- and 3-thienylalanine, β-2- and 3-furanylalanine, β-2-, 3-, and 4-pyridylalanine, β-(benzothienyl-2- and 3-yl)alanine, β-(1- and 2-naphthyl)alanine, O-alkylated derivates of serine, threonine, or tyrosine, S-alkylated cysteine, the O-sulfate ester of tyrosine, 3,5-diiodotyrosine and the D-isomers of the naturally occurring amino acids. The term "any amino acid" is also intended to encompass those naturally ocurring and non-protein α-amino acids of the formulae 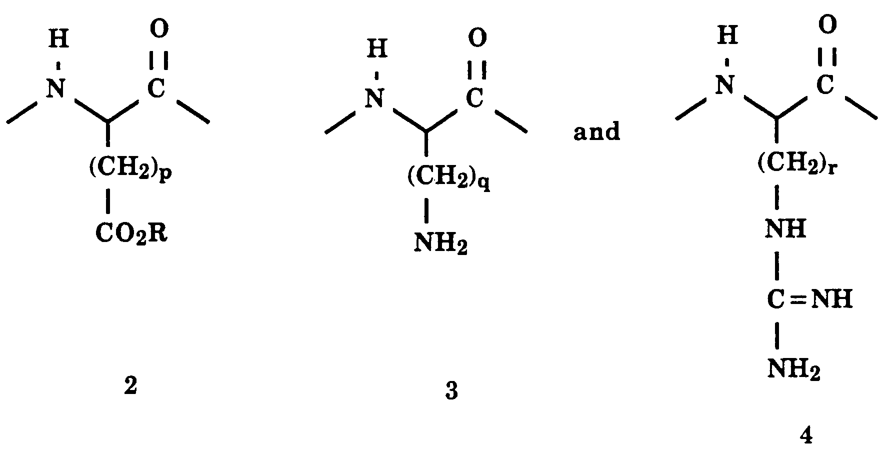
wherein p, q, and r are each independently an integer of from 1 to 5 and wherein R is a hydrogen or a (C₁-C₄)alkyl group.

The term "lipophilic amino acid" includes Tyr, Phe, Leu, Nle, Ile, Val, His and Pro.

The natural amino acids with the exception of glycine, contain a chiral carbon atom. Unless otherwise specifically indicated, the optically active amino acids, referred to herein, are of the L-configuration, including those amino acids depicted by formulae 2, 3, and 4. For example, any of the amino acids of the A₁ or A₁₀ group can be of the D- or L-configuration. As is customary, the structure of peptides written out herein is such that the amino terminal end is on the left side of the chain and the carboxy terminal end is on the right side of the chain. As is also customary when using the three-letter code for the amino acids, a three-letter code begining with an upper case letter indicates the L-confuguration and a three-letter code beginning with a lower-case letter indicates the D-configuration.

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N'-dibenzylethylenediamine, dihydroabietylamine, N-(lower)alkylpiperidine, and any other suitable amine.

As with any generic group of chemical compounds, certain groups are preferred. The use of those peptide derivatives of formula 1 is preferred, wherein
- X: is hydrogen, acetyl, or succinyl.
Also preferred is the use of those formula 1 compounds wherein
- A₁: is 
Thr-Pro-Lys-Pro-Gln-Ser-His-Asn-Asp-Gly-Asp,
-Ser-Thr-Pro-Asn-Pro-Glu-Ser-His-Asn-Asn-Gly-Asp-,
-His-Asn-Asp-Gly-Asp-,
-Asn-Asp-Gly-Asp-,
-Asp-Gly-Asp-,
-Gly-Asp-,
-Asp-,
 or a bond;
- A₂: is as defined above wherein R' is -N(H)C(=NH)NH₂, Z is -NH- or a bond, B is -C(=O)-N(H)-,
- A₃: is preferably Phe, β-2- or 3-thienylalanine, Tyr, Trp, Npa, pClPhe or Tyr(Me);
- q,: an integer of from 2 to 4;
- A₄,: Glu;
- A₅,: Glu, Asp, Pro or Ala;
- A₆,: Ile, Leu;
- A₇,: Pro, Sar, D-Ala, Hyp or NMePgl;
- A₈,: Glu, Gln, Asp or Ala;
- A₉,: Glu, Asp or Ala;
- A₁₀,: Pro, Ala-Tyr, Ala-Cha, Tyr-Cha, Tyr-Leu, Ala-Phe, Tyr-Tyr;
- A₁₁,: Glu, glu, Asn, Asp-Glu, Pro, Gln, Ala, a bond, D-Lys, Lys, asp or Orn; and
- Y,: OH or NH₂.

Also preferred is the use of those compounds of Formula 1 wherein
- X: is hydrogen;
- A₁: is a bond;
- A₂: is of the second represented structure and Z is a bond, q is O, and R' and P are as defined above;
- A₃: is preferably Phe, β-2- or 3-thienylalanine, Tyr, Trp, Npa, pClPhe or Tyr(Me);
- q,: an integer of from 2 to 4;
- A₄,: Glu;
- A₅,: Glu, Asp, Pro or Ala;
- A₆,: Ile, Leu;
- A₇,: Pro, Sar, D-Ala, Hyp or NMePgl;
- A₈,: Glu, Gln, Asp or Ala;
- A₉,: Glu, Asp or Ala;
- A₁₀,: Pro, Ala-Tyr, Ala-Cha, Tyr-Cha, Tyr-Leu, Ala-Phe, Tyr-Tyr;
- A₁₁,: Glu, glu, Asn, Asp-Glu, Pro, Gln, Ala, a bond, D-Lys, Lys, asp or Orn; and
- Y,: OH or NH₂.

Especially preferred is the use of those peptide derivatives of formula 1 wherein either X is succinyl or hydrogen and A₁ is Gly-Asp or Asp or X is succinyl and A₁ is a bond and wherein
- Z,: is -NH- or a bond;
- R': is H₂NC(=NH)NH-;
- q,: 3;
- A₃,: Phe, Tyr, Tyr(Me) or Trp;;
- A₄,: Glu;
- A₅,: Glu or Pro;
- A₆,: Ile;
- A₇,: Pro;
- A₈,: Glu;
- A₉: Glu or Asp;
- A₁₀,: Tyr-Leu, Ala-Tyr, Tyr-Tyr, Ala-Phe, Ala-Cha or Pro;
- A₁₁,: Gln; Asp; Pro; a bond; D-Asp, D-Lys, glu or -Asp-Glu; and
- Y,: OH or NH₂.

Also especially preferred is the use of those peptide derivatives of formula 1 wherein
- X: is hydrogen;
- A₁: is a bond;
- A₂: is the second represented structure and Z is a bond, q is O, P is paraphenylene or 1,4-cyclohexadiyl, and R' is -NH₂ or -N(H)C(=NH)NH₂;
- A₃: is Phe or Trp;
- A₄: is Glu or Pro;
- A₅: is Glu or Pro;
- A₆: is Ile;
- A₇: is Pro;
- A₈: is Glu'
- A₉: is Glu or Asp;
- A₁₀: is Ala or Cha;
- A₁₁: is glu; and
- Y: is OH or NH₂.

The proteins of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of an automated peptide sythesizer. In this procedure an α-amino protected amino acid is bound to a resin support. The resin support employed can be any suitable resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced α-amino protected amino acid.

An example of a hydroxymethyl resin is described by Bodanszky, et al., Chem. Ind. (London) 38, 1597-98 (1966). A chloromethylated resin is commercially available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and α-chlorobutyryl; (2) aromatic urethan type protecting groups such as benzyloxycarbonyl and substituted benzyloxycarbonyl, such as p-chlorobenzyloxycarbonyl, p-nitrobenzyloxycarbonyl p-bromobenzyloxycarbonyl, p-methoxybenzyloxycarbonyl, 1-(p-biphenylyl)-1-methylethoxycarbonyl, α, α-dimethyl-3,5-dimethoxybenzyloxycarbonyl and benzhydryloxycarbonyl; (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thio urethan type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl.

The selection of an appropriate coupling reagent is within the skill of the art. A particularly suitable coupling reagent where the amino acid to be added is Gln, Asn or Arg is N,N'-diisopropylcarbodiimide and 1-hydroxybenzotriazole. The use of these reagents prevents nitrile and lactam formation. Other coupling agents are (1) carbodiimides (e.g., N,N'-dicyclohexylcarbodiimide and N-ethyl-N'-(γ-dimethylaminopropylcarbodiimide); (2) cyanamides (e.g., N,N-dibenzylcyanamide); (3) ketenimines; (4) isoxazolium salts (e.g., N-ethyl-5-phenyl-isoxazolium-3'-sulfonate; (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in dilute aqueous hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

These groups can be removed by procedures well known in the art. Typically protecting group removal is done after the peptide chain synthesis is complete but the protecting groups can be removed at any other appropriate time.

The antiplatelet dose of a peptide analog of this invention is from 0.2 mg/kg to 250 mg/kg of patient body weight per day depending on the patient, the severity of the thromobotic condition to be treated and the peptide analog selected. The suitable dose for a particular patient can be readily determined. Preferably from 1 to 4 daily doses would be administered typically with from 5 mg to 100 mg of active compound per dose.

Antiplatelet therapy is indicated for the prevention of recurrence of myocardial infarction and stroke. as well as other disease conditions associated with platelet aggregation. Those experienced in this field are readily aware of the circumstances requiring anticoagulant and antiplatelet therapy. The term "patient" used herein is taken to mean mammals such as primates, including humans, sheep, horses, cattle, pigs, dogs, cats, rats and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containg a peptide derivative of this invention in a spray or dry powder form.

For parenteral administration the pharmaceutical compositions may be such that the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The pharmaceutical compositions may be such that the compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

### EXAMPLES

This invention is illustrated by the following, nonlimiting examples.

### EXAMPLE 1

### Preparation of 5GP-Gly-Asp-Trp-Glu-Pro-Ile-Pro-Glu-Glu-Ala-Cha-glu-OH

The peptide was synthesized by solid-phase methods using 0.1 mmol of a 0.66 mmol/g Boc-(Bzl)D-Glu- resin. Double symmetrical anhydride couplings were performed with 2.0 mmol Nα-Boc-amino acid (Peptides International). The side chain protection utilized was: Asp(Chx), Trp(CHO), Glu(Bzl). Upon completion of the synthesis the Nα-Boc protection was removed with 50% trifluoroacetic acid in methylene chloride. The resin was washed three times with methylene chloride, neutralized with three washings of 10% diisopropylethylamine in methylene chloride, washed three times with methylene chloride, and dried *in vacuo*. The peptide was deprotected and cleaved from the resin with HF containing 2% anisole at 0°C, for 35 min. The HF was removed *in vacuo* at 0°C, the peptide precipitated with ethyl ether, extracted from the resin with 30% aqueous acetic acid and lyophilized.

Half of this crude 5-AP analog was treated with O-methylisourea at 5°C for 16 hours to yield the crude 5-GP analog.

The peptide was purified by desalting on a 92 x 2.6 cm Sephadex G-15 column in 5% aqueous acetic acid and lyophilized. Preparative HPLC was performed on a C¹⁸ Vydac 218TP1010 (250 x 10 mm) column with 24% acetonitrile in 0.1% aqueous trifluoroacetic acid at 5 ml/min. The major peak was collected and lyophilized. Homogeneity was determined by HPLC and TLC.

In the same manner, the peptides of the following example 2-6 were prepared.

### EXAMPLE 2

### 5AP-Gly-Asp-Trp-Glu-Glu-Ile-Pro-Glu-Glu-Ala-Cha-glu-OH

### EXAMPLE 3

### EXAMPLE 3

### 5AP-Gly-Asp-Tyr(OCH₃)-Glu-Pro-Ile-Pro-Glu-Glu-Ala-Cha-glu-OH

### EXAMPLE 4

### Suc-Tyr-Glu-Pro-Ile-Pro-Arg-Gly-Asp-Phe-glu-OH

### EXAMPLE 5

### 5GP-Gly-Asp-Tyr(OCH₃)-Glu-Pro-Ile-Pro-Glu-Glu-Ala-Cha-glu-OH

### EXAMPLE 6

### 4-Aminomethylbenzoyl-Asp-Trp-Glu-Pro-Ile-Pro-Glu-Glu-Ala-Cha-glu-OH

### EXAMPLE 7

### 4-Guanidinomethylbenzoyl-Asp-Trp-Glu-Pro-Ile-Pro-Glu-Glu-Ala-Cha-glu-OH

### EXAMPLE 8

### 4-Guanidinomethylcyclohexylcarbonyl-Asp-Trp-Glu-Pro-Ile-Pro-Glu-Glu-Ala-Cha-glu-OH

### EXAMPLE 9

### 4-Aminomethylcyclohexylcarbonyl-Gly-Asp-Phe-Glu-Pro-Ile-Pro-Glu-Glu-Ala-Cha-glu-OH

### EXAMPLE 10

### 4-Guanidinomethylcyclohexylcarbonyl-Gly-Asp-Phe-Glu-Pro-Ile-Pro-Glu-Glu-Ala-Cha-glu-OH

The peptides of examples 1 - 12 have the following properties:

| EXAMPLE No. | Amino Acids Analysis (6N HCl Hydrolysis; 24 Hrs at 105°C) | | | | | | |
|---|---|---|---|---|---|---|---|
| | B | Z | Arg | Pro | Ala | Gly | Ile |
| 1 | 0.64 | 4.06 | 1.07 | 2.01 | 1.01 | 0.96 | 0.96 |
| 2 | 0.60 | 4.07 | 1.08 | 1.85 | 1.08 | 0.99 | 0.84 |
| 3 | 0.98 | 4.09 | 1.04 | 1.95 | 1.04 | 0.98 | 0.96 |
| 4 | 1.02 | 4.09 | 0.97 | 1.97 | 0.97 | 0.98 | 1.01 |
| 5 | 0.99 | 1.96 | 1.02 | 1.98 | 1.02 | 0.99 | 0.97 |

| Physical Characteristics | |
|---|---|
| EXAMPLE NO. | FAB-MS (M+H) |
| 1 | 1564.5 |
| 2 | 1523.3 |
| 3 | 1514.1 |
| 4 | 1322.9 |
| 5 | 1556.4 |

| EXAMPLE NO. | Bovine Antithrombin IC₅₀ (µM) | Dog Antiplatelet IC₅₀ (µM) |
|---|---|---|
| 1 | 1.6 | 6 |
| 2 | 1.7 | 250 |
| 3 | 5.4 | 280 |
| 4 | 15 | 42 |
| 5 | 5.9 | 10 |
| 6 | 2.1 | |
| 7 | 1.3 | |
| 8 | 2.3 | |

## Claims

1. The use of a peptide analog of the formula 1
X-A₁A₂A₃A₄A₅A₆A₇A₈A₉A₁₀A₁₁ Y (1)
wherein
X is an amino terminal residue selected from hydrogen, one or two alkyl groups of from 1 to 6 carbon atoms, one or two acyl groups of from 2 to 10 carbon atoms, carbobenzyloxy, H₂NC(NH)-, or t-butyloxy carbonyl group;
A₁ is a bond or is a peptide containing from 1 to 11 residues of any amino acid;
A₂ is a group of the formula 6 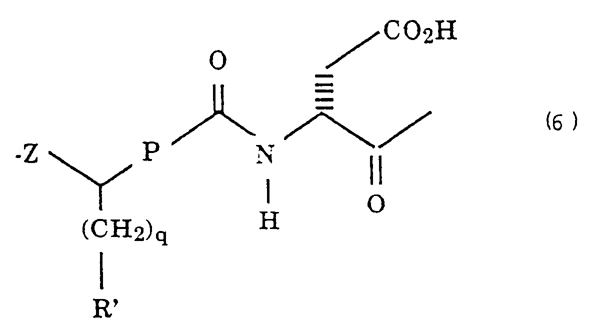
wherein
Z is a bond or a -NH- or N(C₁-C₄alkyl)-group;
q is O or an integer of from 1 to 5;
R' is -NH₂ or -N(H)C(=NH)NH₂;
P is an ortho-, meta-, or para- phenylene or a 1,2-, 1,3-, or 1,4-cyclohexadiyl;
A₃ is Phe, SubPhe, β-(2- and 3-thienyl)alanine, β-(2-and 3-furanyl)alanine, β-(2-, 3-, and 4-pyridyl)alanine, β-(benzothienyl-2- and 3-yl)alanine, β-(1- and 2-naphthyl)alanine, Tyr or Trp;
A₄ is Glu, Asp, Ser(OSO₃H), Ser(OPO₃H), hSer(OSO₃H), cysteic acid or homocysteic acid;
A₅ is any amino acid;
A₆ is Ile, Val, Leu, Nle, or Phe;
A₇ is Pro, Hyp, 3,4-denydroPro, thiazolidine-4-carboxylate, Sar, NMePgl or D-Ala;
A₈ is any amino acid;
A₉ is any amino acid;
A₁₀ is a lipophilic amino acid selected from Tyr, Trp, Phe, Leu, Nle, Ile, Val, Cha and Pro or is a dipeptide containing at least one of these lipophilic amino acids;
A₁₁ is a bond or is a peptide fragment containing from one to five residues of any amino acid; and
Y is a carboxy terminal residue selected from OH, C₁-C₆ alkoxy, amino, mono- or di-(C₁-C₄) alkyl substituted amino, or benzylamino;
or a pharmcaeutically acceptable salt thereof for the preparation of a pharmaceutical composition for use in treating conditions associated with platelet aggregation.

2. The use of claim 1 wherein A₃ is Phe, β-(2-or 3-thienyl)alanine, or Tyr.

3. The use of claim 1 wherein A₄ is Glu.

4. The use of claim 1 wherein A₅ is Glu, Ala, or Pro.

5. The use of claim 1 wherein A₆ is Ile.

6. The use of claim 1 wherein A₇ is Pro.

7. The use of claim 1 wherein A₈ is Glu or Ala.

8. The use of claim 1 wherein A₉ is Glu or Asp.

9. The use of claim 1 wherein A₁₀ is Tyr-Leu, Ala-Tyr, or Ala-Cha.

10. The use of claim 1 wherein A₁₁ is Gln, Asp, Pro, a bond, Asn, Asp-Glu, Glu, Ala, D-Lys, Lys, D-Asp, D-Glu or Orn.

11. The use of claim 1 wherein X is H, acetyl, or succinyl.

12. The use of claim 1 wherein Y is OH or NH₂.

13. The use of the peptide analog 5GP-Gly-Asp-Trp-Glu-Pro-Ile-Pro-Glu-Gly-Ala-Cha-glu-OH, or a pharmaceutically acceptable salt thereof, for the preparation of a pharmaceutical composition for use in treating conditions associated with platelet aggregation.

14. The use of the peptide analog 5AP-Gly-Asp-Trp-Glu-Glu-Ile-Pro-Glu-Glu-Ala-Cha-glu-OH, or a pharmaceutically acceptable salt thereof, for the preparation of a pharmaceutical composition for use in treating conditions associated with platelet aggregation.

15. The use of the peptide analog 5AP-Gly-Asp-Tyr(Me)-Glu-Pro-Ile-Pro-Glu-Glu-Ala-Cha-glu-OH, or a pharmaceutically acceptable salt thereof, for the preparation of a pharmaceutical composition for use in treating conditions associated with platelet aggregation.

16. The use of the peptide analog 5GP-Gly-Asp-Tyr(Me)-Glu-Pro-Ile-Pro-Glu-Glu-Ala-Cha-glu-OH, or a pharmaceutically acceptable salt thereof, for the preparation of a pharmaceutical composition for use in treating conditions associated with platelet aggregation.

17. The use of claim 1 wherein the peptide analog is 4-Aminomethylbenzoyl-Asp-Trp-Glu-Pro-Ile-Pro-Glu-Glu-Ala-Cha-glu-OH.

18. The use of claim 1 wherein the peptide analog is 4-Guanidinomethylbenzoyl-Asp-Trp-Glu-Pro-Ile-Pro-Glu-Glu-Ala-Cha-glu-OH.

19. The use of claim 1 wherein the peptide analog is 4-Guanidinomethylcyclohexylcarbonyl-Asp-Trp-Glu-Pro-Ile-Pro-Glu-Glu-Ala-Cha-glu-OH.

20. The use of claim 1 wherein the peptide analog is 4-Aminomethylcyclohexylcarbonyl-Gly-Asp-Phe-Glu-Pro-Ile-Pro-Glu-Glu-Ala-Cha-glu-OH.

21. The use of claim 1 wherein the peptide analog is 4-Guanidinomethylcyclohexylcarbonyl-Gly-Asp-Phe-Glu-Pro-Ile-Pro-Glu-Glu-Ala-Cha-glu-OH.

22. A method for preparing a pharmaceutical composition for use in the treatment of conditions associated with platelet aggregation which comprises combining an effective amount of a peptide as defined in any of claims 1 to 21, or a pharmaceutically acceptable salt thereof, with a pharmaceutically acceptable carrier.

## Patentansprüche

1. Verwendung eines Peptidanalogs der Formel 1
X-A₁A₂A₃A₄A₅A₆A₇A₈A₉A₁₀A₁₁-Y (1)
wobei
X ein aminoterminaler Rest ist, ausgewählt aus Wasserstoff, einer oder zwei Alkylgruppen mit 1 bis 6 Kohlenstoffatomen, einer oder zwei Acylgruppen mit 2 bis 10 Kohlenstoffatomen, Carbobenzyloxy, H₂NC(NH)- oder t-Butyloxycarbonylgruppen;
A₁ eine Bindung oder ein Peptid ist, das 1 bis 11 Reste irgendeiner Aminosäure enthält;
A₂ eine Gruppe der Formel 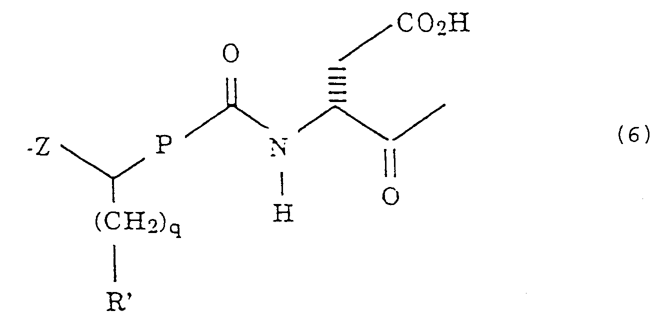 ist, worin
Z eine Bindung oder eine -NH-Gruppe oder ein -N(C₁-C₄-Alkyl)-rest ist;
q 0 oder eine ganze Zahl von 1 bis 5 ist;
R' -NH₂ oder -N(H)C(=NH)NH₂ ist;
P ein ortho-, meta- oder para-Phenylen oder ein 1,2-, 1,3- oder 1,4-Cyclohexadiyl ist;
A₃ Phe, SubPhe, β-(2- und 3-Thienyl)alanin, β-(2- und 3-Furanyl)alanin, β-(2-, 3- und 4-Pyridyl)alanin, β-(Benzothlenyl-2- und 3-yl)alanin, β-(1- und 2-Naphthyl)alanin, Tyr oder Trp ist;
A₄ Glu, Asp, Ser(OSO₃H), Ser(OPO₃H), hSer(OSO₃H), Cysteinsäure oder Homocysteinsäure ist;
A₅ irgendeine Aminosäure ist;
A₆ Ile, Val, Leu, Nle oder Phe ist;
A₇ Pro, Hyp, 3,4-dehydroPro, Thiazolidin-4-carboxylat, Sar, NMePgl oder D-Ala ist;
A₈ irgendeine Aminosäure ist;
A₉ irgendeine Aminosäure ist;
A₁₀ eine lipophile Aminosäure, ausgewählt aus Tyr, Trp, Phe, Leu, Nle, Ile, Val, Cha und Pro oder ein Dipeptid ist, das mindestens eine dieser lipophilen Aminosäuren enthält;
A₁₁ eine Bindung oder ein Peptidfragment ist, das ein bis fünf Reste irgendeiner Aminosäure enthält; und
Y ein carboxyterminaler Rest ist, ausgewählt aus einem OH-, C₁-C₆-Alkoxy-, Amino-, mono- oder di(C₁-C₄)alkylsubstituierten Amino-, oder Benzylaminorest;
oder ein pharmazeutisch verträgliches Salz davon zur Herstellung eines Arzneimittels zur Verwendung in der Behandlung von Krankheiten, die mit Blutplättchenaggregation verbunden sind.

2. Verwendung nach Anspruch 1, wobei A₃ Phe, β-(2- oder 3-Thienyl)alanin oder Tyr ist.

3. Verwendung nach Anspruch 1, wobei A₄ Glu ist.

4. Verwendung nach Anspruch 1, wobei A₅ Glu, Ala oder Pro ist.

5. Verwendung nach Anspruch 1, wobei A₆ Ile ist.

6. Verwendung nach Anspruch 1, wobei A₇ Pro ist.

7. Verwendung nach Anspruch 1, wobei A₈ Glu oder Ala ist.

8. Verwendung nach Anspruch 1, wobei A₉ Glu oder Asp ist.

9. Verwendung nach Anspruch 1, wobei A₁₀ Tyr-Leu, Ala-Tyr oder Ala-Cha ist.

10. Verwendung nach Anspruch 1, wobei A₁₁ Gln, Asp, Pro, eine Bindung, Asn, Asp-Glu, Glu, Ala, D-Lys, Lys, D-Asp, D-Glu oder Orn ist.

11. Verwendung nach Anspruch 1, wobei X H, Acetyl oder Succinyl ist.

12. Verwendung nach Anspruch 1, wobei Y OH oder NH₂ ist.

13. Verwendung des Peptidanalogs
5GP-Gly-Asp-Trp-Glu-Pro-Ile-Pro-Glu-Glu-Ala-Cha-glu-OH
oder eines pharmazeutisch verträglichen Salzes davon zur Herstellung eines Arzneimittels zur Verwendung in der Behandlung von mit Blutplättchenaggregation verbundenen Krankheiten.

14. Verwendung des Peptidanalogs
5AP-Gly-Asp-Trp-Glu-Glu-Ile-Pro-Glu-Glu-Ala-Cha-glu-OH
oder eines pharmazeutisch verträglichen Salzes davon zur Herstellung eines Arzneimittels zur Verwendung in der Behandlung von mit Blutplättchenaggregation verbundenen Krankheiten.

15. Verwendung des Peptidanalogs
5AP-Gly-Asp-Tyr(Me)-Glu-Pro-Ile-Pro-Glu-Glu-Ala-Cha-glu-OH
oder eines pharmazeutisch verträglichen Salzes davon zur Herstellung eines Arzneimittels zur Verwendung in der Behandlung von mit Blutplättchenaggregation verbundenen Krankheiten.

16. Verwendung des Peptidanalogs
5GP-Gly-Asp-Tyr(Me)-Glu-Pro-Ile-Pro-Glu-Glu-Ala-Cha-glu-OH
oder eines pharmazeutisch verträglichen Salzes davon zur Herstellung eines Arzneimittels zur Verwendung in der Behandlung von mit Blutplättchenaggregation verbundenen Krankheiten.

17. Verwendung nach Anspruch 1, wobei das Peptidanalog
4-Aminomethylbenzoyl-Asp-Trp-Glu-Pro-Ile-Pro-Glu-Glu-Ala-Cha-glu-OH ist.

18. Verwendung nach Anspruch 1, wobei das Peptidanalog
4-Guanidinomethylbenzoyl-Asp-Trp-Glu-Pro-Ile-Pro-Glu-Glu-Ala-Cha-glu-OH ist.

19. Verwendung nach Anspruch 1, wobei das Peptidanalog
4-Guanidinomethylcyclohexylcarbonyl-Asp-Trp-Glu-Pro-Ile-Pro-Glu-Glu-Ala-Cha-glu-OH ist.

20. Verwendung nach Anspruch 1, wobei das Peptidanalog
4-Aminomethylcyclohexylcarbonyl-Gly-Asp-Phe-Glu-Pro-Ile-Pro-Glu-Glu-Ala-Cha-glu-OH ist.

21. Verwendung nach Anspruch 1, wobei das Peptidanalog
4-Guanidinomethylcyclohexylcarbonyl-Gly-Asp-Phe-Glu-Pro-Ile-Pro-Glu-Glu-Ala-Cha-glu-OH ist.

22. Verfahren zur Herstellung eines Arzneimittels zur Verwendung in der Behandlung von Krankheiten, die mit Blutplättchenaggregation verbunden sind, umfassend die Kombination einer wirksamen Menge eines in einem der Ansprüche 1 bis 21 definierten Peptids oder eines pharmazeutisch verträglichen Salzes davon mit einem pharmazeutisch verträglichen Träger.

## Revendications

1. Utilisation d'un analogue de peptide de formule 1
X-A₁A₂A₃A₄A₅A₆A₇A₈A₉A₁₀A₁₁-Y (1)
dans laquelle X représente un résidu amino-terminal choisi parmi un atome d'hydrogène, un ou deux groupes alkyle de 1 à 6 atomes de carbone, un ou deux groupes acyle de 2 à 10 atomes de carbone, un groupe carbobenzyloxy, H₂NC(NH)-, ou un groupe t-buryloxycarbonyle;
A₁ représente une liaison ou un peptide contenant de 1 à 11 résidus aminoacides quelconques;
A₂ représente un groupe de formule (6) 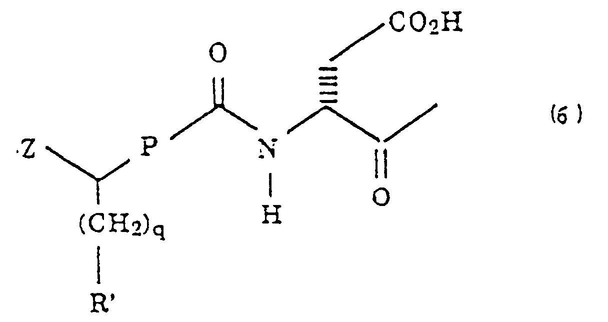 dans laquelle Z représente une liaison ou un groupe -NH- ou -N(alkyle en C₁₋₄);
q vaut 0 ou représente un nombre entier compris entre 1 et 5;
R' représente -NH₂ ou -N(H)C(=NH)NH₂;
P représente un groupe ortho-, méta- ou para-phénylène ou 1,2-, 1,3- ou 1,4-cyclohexadiyle;
A₃ représente Phe, SubPhe, β-(2- et 3-thiényl)alanine, β-(2- et 3-furanyl)alanine, β-(2-, 3- et 4-pyridyl)alanine, β-(benzothiényl-2- et 3-yl)alanine, β-(1-et 2-naphtyl)alanine, Tyr ou Trp;
A₄ représente Glu, Asp, Ser(OSO₃H), Ser(OPO₃H), hSer(OSO₃H), l'acide cystéique ou l'acide homocystéique;
A₅ représente un acide aminé quelconque;
A₆ représente Ile, Val, Leu, Nle ou Phe;
A₇ représente Pro, Hyp, 3,4-déhydroPro, thiazolidine-4-carboxylate, Sar, NMePgl ou D-Ala;
A₈ représente un acide aminé quelconque;
A₉ représente un acide aminé quelconque;
A₁₀ représente un acide aminé lipophile choisi parmi Tyr, Trp, Phe, Leu, Nle, Ile, Val, Cha et Pro ou un dipeptide contenant au moins un de ces acides aminés lipophiles;
A₁₁ représente une liaison ou un fragment peptidique contenant de un à cinq résidus aminoacides quelconques ; et
Y représente un résidu carboxy-terminal choisi parmi OH, un groupe alcoxy en C₁₋₆, amino, amino substitué par un groupe mono- ou di(alkyle en C₁₋₄), ou benzylamino;
ou l'un de ses sels acceptable du point de vue pharmaceutique pour la préparation d'une composition pharmaceutique destinée à être utilisée dans le traitement d'états associés à l'agrégation des plaquettes.

2. Utilisation de la revendication 1, dans laquelle A₃ représente Phe, β-(2- ou 3-thiényl)alanine, ou Tyr.

3. Utilisation de la revendication 1, dans laquelle A₄ représente Glu.

4. Utilisation de la revendication 1, dans laquelle A₅ représente Glu, Ala ou Pro.

5. Utilisation de la revendication 1, dans laquelle A₆ représente Ile.

6. Utilisation de la revendication 1, dans laquelle A₇ représente Pro.

7. Utilisation de la revendication 1, dans laquelle A₈ représente Glu ou Ala.

8. Utilisation de la revendication 1, dans laquelle A₉ représente Glu ou Asp.

9. Utilisation de la revendication 1, dans laquelle A₁₀ représente Tyr-Leu, Ala-Tyr, ou Ala-Cha.

10. Utilisation de la revendication 1, dans laquelle A₁₁ représente Gln, Asp, Pro, une liaison, Asn, Asp-Glu, Glu, Ala, D-Lys, Lys, D-Asp, D-Glu, ou Orn.

11. Utilisation de la revendication 1, dans laquelle X représente H, un groupe acétyle ou succinyle.

12. Utilisation de la revendication 1, dans laquelle Y représente OH ou NH₂.

13. Utilisation de l'analogue de peptide 5GP-Gly-Asp-Trp-Glu-Pro-Ile-Pro-Glu-Glu-Ala-Cha-glu-OH, ou de l'un de ses sels acceptables du point de vue pharmaceutique, pour la préparation d'une composition pharmaceutique destinée à une utilisation dans le traitement d'états associés à l'agrégation des plaquettes.

14. Utilisation de l'analogue de peptide 5AP-Gly-Asp-Trp-Glu-Glu-Ile-Pro-Glu-Glu-Ala-Cha-glu-OH, ou de l'un de ses sels acceptables du point de vue pharmaceutique, pour la préparation d'une composition pharmaceutique destinée à une utilisation dans le traitement d'états associés à l'agrégation des plaquettes.

15. Utilisation de l'analogue de peptide 5AP-Gly-Asp-Tyr(Me)-Glu-Pro-Ile-Pro-Glu-Glu-Ala-Cha-glu-OH, ou de l'un de ses sels acceptables du point de vue pharmaceutique pour la préparation d'une composition pharmaceutique destinée à une utilisation dans le traitement d'états associés à l'agrégation des plaquettes.

16. Utilisation de l'analogue de peptide 5GP-Gly-Asp-Tyr(Me)-Glu-Pro-Ile-Pro-Glu-Glu-Ala-Ala-glu-OH, ou de l'un de ses sels acceptables du point de vue pharmaceutique pour la préparation d'une composition pharmaceutique destinée à une utilisation dans le traitement d'états associés à l'agrégation des plaquettes.

17. Utilisation de la revendication 1, dans laquelle l'analogue de peptide est 4-aminométhylbenzoyl-Asp-Trp-Glu-Pro-Ile-Pro-Glu-Glu-Ala-Cha-glu-OH.

18. Utilisation de la revendication 1, dans laquelle l'analogue de peptide est 4-guanidinométhylbenzoyl-Asp-Trp-Glu-Pro-Ile-Pro-Glu-Glu-Ala-Cha-glu-OH.

19. Utilisation de la revendication 1, dans laquelle l'analogue de peptide est 4-guanidinométhylcyclohexylcarbonyl-Asp-Trp-Glu-Pro-Ile-Pro-Glu-Glu-Ala-Cha-glu-OH

20. Utilisation de la revendication 1, dans laquelle l'analogue de peptide est 4-aminométhylcycloherylcarbonyl-Gly-Asp-Phe-Glu-Pro-Ile-Pro-Glu-Glu-Ala-Cha-glu-OH.

21. Utilisation de la revendication 1, dans laquelle l'analogue de peptide est 4-guanidinométhylcyclohexylcarbonvl-Gly-Asp-Phe-Glu-Pro-Ile-Pro-Glu-Glu-Ala-Cha-glu-OH.

22. Procédé de préparation d'une composition pharmaceutique destinée à une utilisation dans le traitement d'états associés à l'agrégation des plaquettes, qui comprend la combinaison d'une quantité efficace d'un peptide tel que défini dans l'une quelconque des revendications 1 à 21, ou de l'un de ses sels acceptables du point de vue pharmaceutique, avec un véhicule acceptable du point de vue pharmaceutique.
